# EUROPEAN PATENT SPECIFICATION

(11) **EP 3 930 715 B1**
(45) Date of publication and mention of the grant of the patent: **08.05.2024**
(21) Application number: 20710607.1
(22) Date of filing: 26.02.2020
(51) Int. Cl.: A61K 31/4412, A61K 31/496, A61K 45/06, A61P 11/00

(54) **SARACATINIB FOR USE IN THE TREATMENT OF IDIOPATHIC PULMONARY FIBROSIS**
SARACATINIB ZUR VERWENDUNG BEI DER BEHANDLUNG VON IDIOPATHISCHER LUNGENFIBROSE
SARACATINIB POUR UTILISATION DANS LE TRAITEMENT DE LA FIBROSE PULMONAIRE IDIOPATHIQUE

(30) Priority: 27.02.2019 US 201962811009 P
(43) Date of publication of application: 05.01.2022
(73) Proprietor: Astrazeneca AB, 151 85 Södertälje (SE); Fraunhofer-Gesellschaft zur Förderung der angewandten Forschung e.V., 80686 München (DE)
(72) Inventor: COUSENS, Leslie, Waltham, Massachusetts 02451 (US); NIVENS, Chad, Waltham, Massachusetts 02451 (US); ESCOTT, Katherine Jane, Cambridge CB2 0AA (GB); MONKLEY, Susan, Mölndal (SE); PRASSE, Antje, München (DE)
(74) Representative: AstraZeneca Intellectual Property
(86) International application number: PCT/IB2020/051642
(87) International publication number: WO 2020/174420

(56) References cited:
- WO-A1-2016/123086
- WO-A1-2018/026442
- LI-FU LI ET AL: "Nintedanib reduces ventilation-augmented bleomycin-induced epithelial-mesenchymal transition and lung fibrosis through suppression of the Src pathway", JOURNAL OF CELLULAR AND MOLECULAR MEDICINE, vol. 21, no. 11, 9 June 2017 (2017-06-09), pages 2937-2949, XP055695805, RO ISSN: 1582-1838, DOI: 10.1111/jcmm.13206
- YILMAZ OZNUR ET AL: "Dasatinib attenuated bleomycin-induced pulmonary fibrosis in mice", GROWTH FACTORS, vol. 33, no. 5-6, 11 November 2015 (2015-11-11), pages 366-375, XP008181486, ISSN: 1029-2292, DOI: 10.3109/08977194.2015.1109511 [retrieved on 2015-11-25]
- ABDALLA MAHA ET AL: "Dasatinib inhibits TGF[beta]-induced myofibroblast differentiation through Src-SRF Pathway", EUROPEAN JOURNAL OF PHARMACOLOGY, ELSEVIER SCIENCE, NL, vol. 769, 6 November 2015 (2015-11-06), pages 134-142, XP029334525, ISSN: 0014-2999, DOI: 10.1016/J.EJPHAR.2015.11.008
- S. HARARI ET AL: "IPF: new insight on pathogenesis and treatment", ALLERGY, vol. 65, no. 5, 1 May 2010 (2010-05-01), pages 537-553, XP055695528, United Kingdom ISSN: 0105-4538, DOI: 10.1111/j.1398-9995.2009.02305.x
- HU MENG ET AL: "Therapeutic targeting of SRC kinase in myofibroblast differentiation and pulmonary fibrosis", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 351, no. 1, 30 September 2014 (2014-09-30), pages 87-95, XP009524969, ISSN: 1521-0103, DOI: 10.1124/JPET.114.216044 [retrieved on 2014-08-27]

## Description

### FIELD

The present disclosure concerns Saracatinib, or a pharmaceutically-acceptable salt thereof, for use in the treatment idiopathic pulmonary fibrosis in a human, as defined in the claims. The disclosure also concerns said Saracatinib, or a pharmaceutically-acceptable salt thereof, in combination with nintedanib or a pharmaceutically acceptable salt thereof for use in the treatment of idiopathic pulmonary fibrosis, in a human, as defined in the claims. The disclosure also concerns a pharmaceutical combination comprising saracatinib or a pharmaceutically acceptable salt thereof, and nintedanib or a pharmaceutically acceptable salt thereof, as defined in the claims.

### BACKGROUND

Remodelling is a normal response to tissue injury and inflammation and is observed in many tissues throughout the body. After resolution of the inflammation and repair of tissue damage, the tissue is generally returned to its original condition. However, excessive uncontrolled tissue repair or the failure to stop remodelling when it is no longer required leads to a condition marked by fibrosis. Fibrosis typically involves the accumulation of extracellular matrix constituents that occur following trauma, inflammation, tissue repair, immunological reactions, cellular hyperplasia or neoplasia. Examples of tissue fibrosis include, but are not limited to, pulmonary fibrosis, renal fibrosis, cardiac fibrosis, cirrhosis and fibrosis of the liver, skin scars and keloids, adhesions, fibromatosis, atherosclerosis, and amyloidosis. Fibrosis often severely compromises the normal function(s) of the organ affected and many fibrotic conditions are, in fact, life-threatening or severely disfiguring. Unfortunately, treatment options for these diseases are limited; risky and expensive procedures such as organ transplantation are often the only possible approaches available.

An example of a particularly severe fibrotic condition with a high unmet clinical need is Idiopathic pulmonary fibrosis (IPF). IPF is a chronic, relentless, and ultimately fatal disorder characterized by scarring (fibrosis) of the lung parenchyma. The disease causes debilitating cough, lung function decline, fatigue and dyspnoea and the need for high-levels of supplemental oxygen limit physical activity and dramatically reduce patient quality of life and independence over time. The median survival of patients with IPF ranges from 2.5 years to 3.5 years, with most patients dying from respiratory failure due to disease progression (Raghu G, et al., Am J Respir Critical Care Med 2015; Vo. 192, No. 2: https://doi.org/10.1164/rccm.201506-1063ST).

The disease pathology of IPF is poorly understood and few drug treatment options exist (Mason D P et al., Ann Thoracic Surgery 84:1121-8, 2007). In 2014, the FDA approved two new antifibrotic drugs for the treatment of IPF, pirfenidone (Esbriet^{®}) and nintedanib (OFEV^{®}). The mechanism of action of pirfenidone has not been fully established. Nintedanib is a tyrosine kinase inhibitor that specifically targets the PDGFR α and β receptor, vascular endothelial growth factor receptor (VEGFR 1-3) and fibroblast growth factor receptor (FGFR 1-3) kinases. Clinical trials and real-world experience demonstrate that, while on average, both drugs slow the rate of decline in lung function, responses are variable, compliance is challenging due to unfavourable safety, and neither drug halts the progressive loss of lung function. Furthermore, neither drug cures IPF, improves symptoms or demonstrates overall survival benefit. Importantly, clinical experience with the two agents has shown that one year after initiation of anti-fibrotic therapy, almost 40% of patients had discontinued treatment with adverse side effects of the gastrointestinal track or skin being the most frequent reason for termination (Corte T, et al., Respiratory research 2015; 16:116 and Xaubet A, et al., Am J Respir Critical Care Med 2003;168(4):431-5). Moreover, treatment with pirfenidone, which is indicated for mild to moderate IPF, requires titration to very high and frequent doses, presenting both safety and patient compliance issues. The recommended daily dose of pirfenidone from day 15 onwards is 2403 mg/day, which requires a patient to take nine Esbriet^{®} capsules per day (three 267 mg capsules three times a day with food). LI-FU LI Et AL ("Nintedanib reduces ventilation-augmented bleomycin-induced epithelial-mesenchymal transition and lung fibrosis through suppression of the Src pathway", JOURNAL OF CELLULAR AND MOLECULAR MEDICINE, vol. 21, no. 11, 9 June 2017 (2017-06-09), pages 2937-2949, DOI: 10.1111/jcmm.13206) disclose that nintedanib is a Src inhibitor and that it can ameliorate pulmonary fibrosis by suppressing the Src pathway. It is further disclosed that nintedanib has been approved for the treatment of idiopathic pulmonary fibrosis.

Despite the US approval of Esbriet^{®} and OFEV^{®} in 2014, IPF remains a chronic, fatal disease that impairs patients' quality of life and drives up health care utilization and costs. Thus, there remains an urgent need for development of more effective therapies that safely and more reliably modify the course of the different forms/stages of IPF to maintain/restore quality of life.

Saracatinib (ADZ0530) is a potent inhibitor of the Src family of tyrosine kinases with high selectivity over other protein kinases involved in signal transduction (Chang YM, et al., Oncogene 2008: 27(49):6365-75 and Greet TO et al., Mol Oncol. 2009; 3(3): 248-61). The compound together with its manufacture are disclosed in PCT patent application WO 01/94341, which concerns the use of quinazoline derivatives for the treatment of tumours. Saracatinib was originally being developed for the treatment of cancer, however, clinical trials failed to show sufficient efficacy in this indication. Saracatinib has also been investigated in Alzheimer's disease (Nygaard et al., Alzheimer's Res Therapeutic, 2015; 7(1): 35). WO2016/123086 describes a method of treating a cardiovascular disease or condition having aberrant protein tyrosine phosphorylation in a subject, comprising administering a low- dosage of a tyrosine kinase inhibitor (such as saracatinib) to a subject in need thereof, wherein the tyrosine kinase inhibitor decreases aberrant levels of tyrosine phosphorylation and improves at least one cardiac function in the subject.

Saracatinib has also been tested in a bleomycin murine lung fibrosis model and found to influence myofibroblast differentiation (Hu M et al., Journal of Pharmacology and Experimental Therapeutics; 2014, 351: 87-95). The bleomycin murine model has the advantage that it is quite easy to perform and does have some similarities in histological alterations seen in IPF. However, whilst numerous agents have been tested and found to exhibit some activity in this disease model, relatively few have been progressed to recapitulate this activity in humans (Moeller A, et al., Int J Biochem Cell Biol. 2008; 40(3): 362-282). Part of the reason for this is that the model has an acute inflammatory component with a later fibrotic phase that does not replicate IPF and is therefore of limited value in respect to providing information about the profile of activity of a compound in clinical use. Many anti-inflammatory mechanisms have demonstrated efficacy in the bleomycin challenge model, but failed to show clinical utility (e.g., tralokinumab, lebrikizumab, SNY program of IL-4/ 13). As acknowledged by Hu et al., whilst bleomycin treatment induces lung fibrosis in rodents, and the resulting fibrosis shares many key features of human lung fibrosis, the bleomycin model does not replicate human IPF and is not a model of progressive fibrosis. Fibrosis tends to only persist 3 to 4 weeks post bleomycin instillation before spontaneously resolving, returning the lungs to near-normal state with minimal fibrosis. In addition to diverging from the human disease by exhibiting spontaneous resolution of fibrosis, the bleomycin model also lacks other cell types, processes and structures critically associated with human disease progression, such as hyperplastic type II alveolar epithelial cells (AECs), bronchiolization and honeycomb cysts. The aspect of slow and reversible progression of IPF in patients is not reproduced in the bleomycin model (Chua F, et al., Am J Respir Cell Molecular Biology 2005; 33(1):9-13) and as such, the bleomycin model has significant limitations in regard to understanding the progressive and irreversible nature of human IPF. This is particularly relevant when the clinical activity of a compound in different disease stages and/or different forms of progressive IPF is to be evaluated.

IPF is recognised as a heterogenous disease that can be characterised by stage and severity. Published studies have suggested that the clinical course of IPF is variable but distinct subgroups of patients do exist, for example in whom fibrosis progresses more rapidly (Ley B et al., Ann Intern Med 2012; 156(10):684-91 and Martinez FJ, et al. 2005; 142(12): 963-7). Recently, Herazo-Maya et al. published a retrospective study identifying a peripheral blood transcriptomic signature predictive of mortality and transplant-free survival in patients with IPF (Herazo-Maya et al. Lancet Respir Med 2017; 5: 857-68). The signature correlates with FVC and, without therapy, is stable over time. Data from a small patient group suggest that with response to therapeutic intervention, the high-risk profile is normalized. In the local lung environment, Prasse et al, have identified in bronchiolar lavage samples from IPF patients a transcriptomic signature that is driven by airway basal cells and is predictive of high versus low risk of mortality or lung transplant (Prasse et al. 2018 https://doi.org/10.1164/rccm.201712-25510C, PMID: 30141961). These results provide important insights into biomarkers of disease progression and response to therapy with the potential to implicate new therapeutic targets. Achieving effective treatment in such different groups of patients is challenging. Regardless of the heterogeneity, neither of the current drugs halts the progressive loss of lung function, reverses disease, or cures IPF.

Thus, it is apparent that there remains an urgent need for development of more effective therapies that safely and more reliably modify the course of the different forms and stages of fibrotic diseases and conditions.

Surprisingly, it has been shown by this disclosure that Src kinase inhibitor saracatinib operates through a distinct mechanism of action and offer particular benefits over existing medicines for the treatment of fibrotic conditions. For example, the present disclosure has demonstrated that saracatinib targets multiple drivers of fibrosis pathology and is particularly effective in the treatment of idiopathic pulmonary fibrosis (including progressive forms and/or different stages of idiopathic pulmonary fibrosis) and/or offer a safer and better-tolerated therapy for such conditions when compared to existing medicines such as pirfenidone and nintedanib.

Furthermore, applicants have also found that Src kinase inhibitor saracatinib is surprisingly effective when used in combination with at least one additional therapeutic agent, e.g., an anti-fibrotic, for the treatment of idiopathic pulmonary fibrosis, in a warm-blooded animal such as a human.

### SUMMARY

The Src kinase inhibitor Saracatinib or a pharmaceutically acceptable salt thereof of the current disclosure is surprisingly effective in the treatment of certain Idiopathic Pulmonary Fibrosis (IPF) conditions by mechanisms distinct from conventional methods of treatment. In one embodiment, the IPF is characterized by persistent, debilitating abnormal formation of lesions or scars determined by high-resolution computed tomography (HRCT) or biopsy. In another embodiment, the IPF is characterized by abnormal collagen deposition. The present disclosure thus relates to Saracatinib or a pharmaceutically acceptable salt thereof for use in treating such IPF in a human patient.. In one embodiment, the Src kinase inhibitor Saracatinib or a pharmaceutically acceptable salt thereof at its safe and tolerable dose is selective for inhibiting Src kinase with a clinically relevant potency. In one embodiment, the IPF are characterized by epithelial to mesenchymal transition (EMT). In one embodiment, the IPF is characterised by increased expression of Collagen-I and/or MMP-9 and/or TIMP-1. In one embodiment, IPF is characterized by increased expression of sVEGF and/or sIL-8 and/or sIL-6. In one embodiment, the IPF is characterized by decreased expression of VCAM-1. In one embodiment, the IPF is characterized by extra cellular matrix (ECM) formation.

In another aspect, the present disclosure relates to Saracatinib, or a pharmaceutically acceptable salt thereof, for use in treatment of a human patient with idiopathic pulmonary fibrosis (IPF), wherein the Saracatinib or a pharmaceutically acceptable salt thereof is administered in combination with nintedanib or a pharmaceutically acceptable salt thereof.

The present disclosure also relates to a pharmaceutical composition comprising Saracatinib or a pharmaceutically acceptable salt thereof together with a pharmaceutically suitable carrier for use in the treatment of IPF as described herein in a human patient.

The present disclosure further relates to a pharmaceutical combination for the treatment of IPF as described herein, which comprises Saracatinib or a pharmaceutically acceptable salt thereof in combination with nintedanib or a pharmaceutically acceptable salt thereof.

### DESCRIPTION OF THE DRAWINGS

Embodiments of the disclosure are further described hereinafter with reference to the accompanying drawings, in which the values in relation to pirfenidone and nintedanib alone are provided for reference:
FIG. 1 shows the hydroxyproline levels in mice after bleomycin (Bleo) induced lung injury and fibrosis following treatment with pirfenidone (PIR) at 200 mg/kg BID, nintedanib (NIN) at 30 mg/kg BID and saracatinib at 1 (0530-1), 3 (0530-3) or 10 (0530-10) mg/kg BID.
FIG. 2 shows the % max αSMA levels of human lung fibroblasts treated with saracatinib (circles), nintedanib (triangles) or pirfenidone (squares).
FIG. 3 shows the mean IL-6 levels of human lung fibroblasts treated with saracatinib (circles), nintedanib (triangles) or pirfenidone (squares).
FIG. 4 shows organoid formation of airway basal cells (ABCs) treated with saracatinib, pirfenidone or nintedanib.
FIG. 5 shows a higher-powered image of organoid formation of ABCs treated with saracatinib (SARA), pirfenidone (Pirf) or nintedanib (Nin) at the concentrations indicated.
FIG. 6 shows the optical density/well and organoid counts/well for ABCs treated with saracatinib, pirfenidone or nintedanib.
FIG. 7 shows the DiscoverX assay results indicating relative protein expression (log ratio of compound/vehicle control) for saracatinib (3.3 µM).
FIG. 8 shows the DiscoverX assay results indicating relative protein expression (log ratio of compound/vehicle control) for nintedanib (1.1 µM).
FIG. 9 shows the DiscoverX assay results indicating relative protein expression (log ratio of compound/vehicle control) for the combination of saracatinib and nintedanib compared to saracatinib and nintedanib alone.
FIG. 10 shows the kinase trees of saracatinib (Sara) and nintedanib (Ninte) where each circle marks a kinase inhibited by the respective molecule, and the size of the circle represents potency of this inhibition.

### DESCRIPTION OF ILLUSTRATIVE EMBODIMENTS

### Definitions

As used herein, the phrase "effective amount" means an amount of a Src kinase inhibitor or composition comprising a Src kinase inhibitor which is sufficient to significantly and positively modify the symptoms and/or conditions to be treated (e.g., provide a positive clinical response). The effective amount of an active ingredient(s) for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

As used herein, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Reference to a particular numerical value includes at least that particular value, unless the context clearly dictates otherwise. When a range of values is expressed, another embodiment includes from the one particular value and/or to the other particular value. Further, reference to values stated in ranges include each and every value within that range. All ranges are inclusive and combinable.

It is to be appreciated that certain features of the disclosed Src kinase inhibitors, compositions, and methods which are, for clarity, described herein in the context of separate embodiments, may also be provided in combination in a single embodiment. Conversely, various features of the disclosed Src kinase inhibitors, compositions and methods that are, for brevity, described in the context of a single embodiment, may also be provided separately or in any sub-combination.

As used herein, the singular forms "a," "an," and "the" include the plural.

When values are expressed as approximations, by use of the antecedent "about," it will be understood that the particular value forms another embodiment.

The term "about" when used in reference to numerical ranges, cut-offs, or specific values is used to indicate that the recited values may vary by up to as much as 10% from the listed value. As many of the numerical values used herein are experimentally determined, it should be understood by those skilled in the art that such determinations can, and often times will, vary among different experiments. The values used herein should not be considered unduly limiting by virtue of this inherent variation. Thus, the term "about" is used to encompass variations of ± 10% or less, variations of ± 5% or less, variations of ± 1% or less, variations of ± 0.5% or less, or variations of ± 0.1% or less from the specified value.

As used herein, "treating" and like terms refer to reducing the severity and/or frequency of symptoms, eliminating symptoms and/or the underlying cause of said symptoms, reducing the frequency or likelihood of symptoms and/or their underlying cause, delaying, preventing and/or slowing the progression of fibrosis, such as IPF, and improving or remediating damage caused, directly or indirectly, by the fibrosis, such as IPF. As used herein, treating is intended to embrace prophylaxis as well as therapeutic treatment.

### Src kinase inhibitor

The present disclosure has demonstrated that Src kinase inhibitor Saracatinib or a pharmaceutically acceptable salt thereof offers particular benefits over existing medicines for the treatment of idiopathic pulmonary fibrosis (IPF) as described herein.

In one embodiment, the Src kinase inhibitor is a selective Src kinase inhibitor i.e. the Src kinase inhibitor is selective for the Src family kinases over other protein kinases involves in signal transduction. Suitably, the Src family comprises c-Src, c-Yes, Lck, Lyn, and Fyn and in one particular embodiment, the Src kinase inhibitor is selective for c-Src, c-Yes, Lck, Lyn, and c-Fyn kinases. In one embodiment, the Src kinase inhibitor has an IC₅₀ assay for each of c-Src, c-Yes, Lck, Lyn, and c-Fyn kinases of less than or equal to 100 nM, such as less than or equal to 75, 50, 40, 30, 20, 15, 12 or 10 nM. In one embodiment, the Src kinase inhibitor has an IC₅₀ assay for PDGFRα and/or PDGFRβ of above 1000 nM.

In one embodiment, the Src kinase inhibitor is selective for c-Src kinase and has an IC₅₀ assay for c-Src kinase of less than or equal to 100 nM, such as less than or equal to 75, 50, 40, 30, 20, 15, 12, 10, 8, 6, 4 or about 3 nM. In a further embodiment, the Src kinase inhibitor is selective for c-Yes kinase and has an IC₅₀ assay for c-Yes kinase of less than or equal to 100 nM, such as less than or equal to 75, 50, 40, 30, 20, 15, 12, 10, 8, 6 or 4 nM. In yet a further embodiment, the Src kinase inhibitor is selective for Lck kinase and has an IC₅₀ assay for Lck kinase of less than or equal to 100 nM, such as less than or equal to 75, 50, 40, 30, 20, 12, 10, 6 or 4 nM. In yet a further embodiment, the Src kinase inhibitor is selective for Lyn kinase and has an IC₅₀ assay for Lyn kinase of less than or equal to 100 nM, such as less than or equal to 75, 50, 40, 30, 20, 15, 12, 10, 8, 6 or 5 nM. In yet a further embodiment, the Src kinase inhibitor is selective for c-Fyn kinase and has an IC₅₀ assay for c-Fyn kinase of less than or equal to 100 nM, such as less than or equal to 75, 50, 40, 30, 20, 15, 12 or 10 nM. In yet a further embodiment, the Src kinase inhibitor has an IC₅₀ assay for EGFR of less than or equal to 200 nM, such as less than or equal to 150, 100, 70 or about 66 nM.

In one embodiment, the Src kinase inhibitor has an IC₅₀ assay for PDGFRα and/or PDGFRβ of greater than or equal to 5000 nM, such as greater than or equal to 6000, 7000, 10000 nm.

Tyrosine kinase inhibitors, such as VEGFR inhibitors and PDGFR inhibitors, are associated with toxic effects on various organs such as the heart, lungs, liver, kidneys, thyroid, skin, blood coagulation, gastrointestinal tract and nervous system. Thus, the lack of activity against PDGFR advantageously contributes to a better tolerated Src kinase inhibitor which in turn may result in an improved safety profile and/or improved patient compliance (Li et al. Eur J Clin Pharmacol. 2017 Oct;73(10):1209-1217).

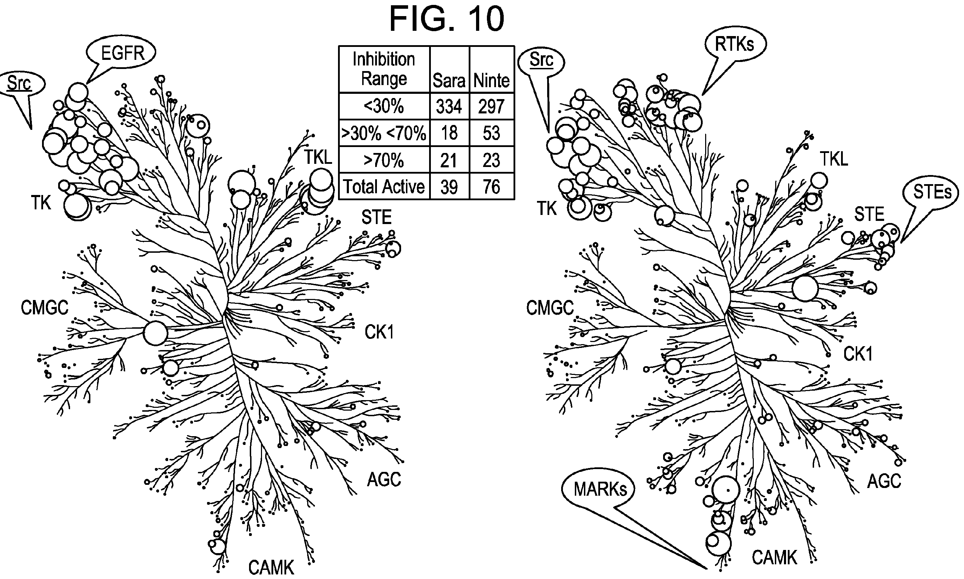

The present disclosure concerns a Src kinase inhibitor that is saracatinib or a pharmaceutically acceptable salt thereof. FIG. 10 shows the kinase trees of saracatinib and nintedanib demonstrating the superior selectivity of saracatinib in a graphic presentation. Saracatinib has the chemical name of N-(5-chlorobenzo[d][1,3]dioxol-4-yl)-7-(2-(4-methylpiperazin-1-yl)ethoxy)-5-((tetrahydro-2H-pyran-4-yl)oxy)quinazolin-4-amine and structure as shown below:

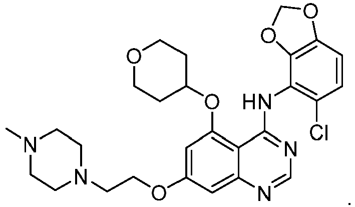

In one embodiment, saracatinib is in form of a fumarate salt. In another embodiment, saracatinib is in form of a difumarate salt. Further details of saracatinib and its analogs, including the preparations thereof, are described in WO 01/94341 published on 13 December 2001 and entitled "Quinazoline Derivatives for the Treatment of Tumors"; and WO 2006/064217, published on 22 June 2006 and entitled "Chemical Process".

A suitable pharmaceutically-acceptable salt of the Src kinase inhibitor is, for example, an acid addition salt or a base salt. Such salts are physiologically non-toxic.

Examples of pharmaceutically acceptable acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, formate, fumarate, glutamate, glycolate, hemisulfate, 2 hydroxyethyl sulfonate, heptanoate, hexanoate, hydrochloride, hydrobromide, hydroiodide, hydroxymaleate, lactate, malate, maleate, methanesulfonate, meglumine, 2 naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p toluenesulfonate), trifluoroacetate, and undecanoate.

Examples of pharmaceutically acceptable base salts include ammonium salts; alkali metal salts such as sodium, lithium and potassium salts; alkaline earth metal salts such as aluminum, calcium and magnesium salts; salts with organic bases such as dicyclohexylamine salts and N methyl D glucamine; and salts with amino acids such as arginine, lysine, ornithine, and so forth. Also, basic nitrogen containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

### Fibrosis

"Fibrosis" or "fibrotic disease or condition", as used herein, refers to the accumulation of extracellular matrix constituents that occurs following trauma, inflammation, tissue repair, immunological reactions, cellular hyperplasia and neoplasia. Examples of tissue fibrosis include, but are not limited to, pulmonary fibrosis, renal fibrosis, cardiac fibrosis, cirrhosis and fibrosis of the liver, skin scars and keloids, adhesions, fibromatosis, atherosclerosis and amyloidosis.

In one aspect, there is provided saracatinib or a pharmaceutically acceptable salt thereof, for use in a method of treating Idiopathic Pulmonary Fibrosis (IPF), comprising administering to the human patient a therapeutically effective amount of saracatinib or a pharmaceutically acceptable salt thereof as described herein. In another aspect, there is provided saracatinib, or a pharmaceutically acceptable salt thereof, as described herein, for use in treating Idiopathic Pulmonary Fibrosis (IPF)in a human patient as described herein. In one embodiment, the saracatinib or pharmaceutically acceptable salt thereof demonstrates clinically relevant potency at its safe and tolerable dose.

Suitably, the IPF is characterized by persistent, debilitating abnormal formation of lesions or scars determined by high-resolution computed tomography (HRCT) or biopsy. In another embodiment, the IPF is characterized by abnormal collagen deposition.

In one embodiment, the IPF is characterised by epithelial to mesenchymal transition (EMT). In another embodiment, the IPF is characterised by increased expression of Collagen-I and/or MMP-9 and/or TIMP-1 in a human patient. In one embodiment, the IPF is characterised by increased expression of Collagen-I, MMP-9 and TIMP-1 in a human patient. In one embodiment, the IPF is characterised by increased expression of Collagen-I and MMP-9 in a human patient. In one embodiment, the IPF is characterised by increased expression of Collagen-I and TIMP-1 in a human patient. In one embodiment, the IPF is characterised by increased expression of MMP-9 and TIMP-1 in a human patient. In another embodiment, the IPF is characterized by extra cellular matrix (ECM) formation.In some embodiments, treatment of a IPF in a human patient includes reducing or inhibiting one or more of: formation or deposition of extracellular matrix proteins; the number of pro-fibrotic cell types (e.g., fibroblast or immune cell numbers); cellular collagen or hydroxyproline content within a fibrotic lesion; expression or activity of a fibrogenic protein; or reducing fibrosis associated with an inflammatory response.
In some embodiments, the IPF is associated with (e.g., is secondary to) a disease (e.g., an infectious disease, an inflammatory disease, an autoimmune disease, a malignant or cancerous disease, and/or a connective disease); a toxin; an insult (e.g., an environmental hazard (e.g., asbestos, coal dust, polycyclic aromatic hydrocarbons), cigarette smoking, a wound); a medical treatment (e.g., surgical incision, chemotherapy or radiation), or a combination thereof.
In some embodiments, the IPF is secondary to a disease, a toxin, an insult, a medical treatment, or a combination thereof. In some embodiments, IPF is associated with one or more of: a disease process such as asbestosis and silicosis; an occupational hazard; an environmental pollutant; cigarette smoking; an autoimmune connective tissue disorders (e.g., rheumatoid arthritis, scleroderma and systemic lupus erythematosus (SLE)); a connective tissue disorder such as sarcoidosis; an infectious disease, e.g., infection, particularly chronic infection; a medical treatment, including but not limited to, radiation therapy, and drug therapy, e.g., chemotherapy (e.g., treatment with as bleomycin, methotrexate, amiodarone, busulfan, and/or nitrofurantoin). In some embodiments, the treatment of IPF of the disclosure is associated with (e.g., secondary to) a cancer treatment, e.g., treatment of a cancer (e.g. squamous cell carcinoma, testicular cancer, Hodgkin's disease with bleomycin).

Suitably, the fibrotic condition, which is characterised by ECM, or increased expression of Collagen-I, MMP-9 and/or TIMP-1, or EMT in a human patient is idiopathic pulmonary fibrosis.

### Idiopathic pulmonary fibrosis

The present disclosure concerns a fibrotic condition that is idiopathic pulmonary fibrosis (IPF). In one particular embodiment, the fibrotic condition is progressive IPF. Progressive IPF may be characterised in respect to the rate of disease progression. For example, patients with rapidly progressive IPF and patients with slowly progressive IPF can be distinguished. Slowly progressive IPF can be defined as IPF where there is a slow progressive decline in lung function and worsening dyspnoea. Often, slowly progressive IPF patients experience a long duration of symptoms before diagnosis and experience a slowly progressive clinical course (Martinez FX et al., Ann Intern Med 2005; 142: 963-967). Slowly progressive IPF can lead to death within several years of diagnosis. Rapidly progressive IPF involves patients displaying a more rapidly progressive clinical course with a shorter duration of symptoms before diagnosis and progression to death.

In one embodiment, there is provided saracatinib or a pharmaceutically acceptable salt thereof, for use in a method of treating an IPF as described herein, comprising administering to the human patient a therapeutically effective amount of saracatinib or a pharmaceutically acceptable salt thereof. In another aspect, there is provided saracatinib, or a pharmaceutically acceptable salt thereof, for use in treating IPF as described herein in a human patient.

The skilled person would be aware that pulmonary function tests can provide some indication of the extent and severity of IPF. For example, Forced Vital Capacity (FVC), which is a measurement of lung function in litres and represents the volume of air in the lungs that can be exhaled following a full inhalation, can provide an indication of the severity of IPF in a patient. FVC is measured in a test known as spirometry which is a specific type of pulmonary function test. Diffusing capacity of the lung for carbon monoxide (DLCO), which represents the extent to which oxygen passes from the air sacs of the lungs into the blood, can also provide an indication of the extent and severity of IPF in a patient. Both FVC and DLCO can be expressed as a percentage of the predicted normal for a person of the same sex, age and height. The skilled person will be aware of methods to determine and use the FVC and DLCO parameters.

In one embodiment, progressive IPF is characterised by an FVC threshold of between 20% and 80% predicted, such as between 50 and 75%, between 50 and 65%, between 50 and 60% or between 50 and 55% predicted. In one embodiment, progressive IPF is characterised by a DLCO of between 20 and 70% predicted, such as between 35 and 55%, between 35 and 45%, or between 35 and 40% predicted.

Disease severity of IPF may be classified as mild, moderate or severe. While there is no standardised definition of mild, moderate or severe IPF, clinical trials have generally employed an FVC threshold of 50-55% predicted and a DLCO threshold of 35-40% predicted to separate mild-to-moderate patients from those with severe disease (M Kolb et al., Eur. Respir Rev 2014; 23: 220-224).

In one embodiment, the IPF is mild, moderate, or mild-to-moderate IPF. In one embodiment, the mild-to-moderate IPF is characterised by an FVC threshold of greater than or equal to 50% predicted, such as 50 and 75% or 50 to 55% predicted and/or a DLCO threshold of greater than or equal to 35% predicted, such as 20 and 70% predicted or 35 to 40% predicted.

In one embodiment, the IPF is severe IPF. In one embodiment, the severe IPF is characterised by FVC threshold of less than 50% predicted, such as less than 45%, 40%, 35%, 30%, 25%, 20%, 10%, or 5% predicted, and/or a DLCO threshold of less than 35% predicted, such as less than 30%, 25%, 20%, 15%, 10%, or 5% predicted.

In one embodiment, progressive IPF is characterised by an annual rate of decline, as measured by FVC, of less than or equal to 0.3 L, such as less than or equal to 0.25L, 0.15 L or 0.12 L. Suitably, the annual rate of decline, as measure by FVC, is between 0.1 L and 0.3 L, such as between 0.13 L and 0.21 L. Suitably, the annual rate of decline, as measured by FVC, is greater than or equal to 0.05 L, such as greater than or equal to 0.08 L, 0.10 L or 0.13 L. In one embodiment, progressive IPF is characterised by a rate of decline in FVC is greater than or equal to 1%, such as greater than or equal to 2%, 4%, 6%, 8%, 10%, 12%, 14%, 16%, 18% or 20%. Suitably, the rate of decline in FVC is less than or equal to 20%, such as less than or equal to 18%, 16%, 14%, 12%,10%, 8%, 6%, 4%, 2% or 1%. Suitably, the rate of decline in FVC is about 5%, such as about 10%, about 15% or about 20%.

In one embodiment, progressive IPF is characterised by a rate of decline in DLCO of greater than or equal to 1%, such as greater than or equal to 2%, 4%, 6%, 8%,10%,12%, 14%, 15%, 16%, 18%, 20%, 22% or 25%. Suitably the rate of decline in DLCO is less than or equal to 25%, such as less than or equal to 22%, 20%, 18%, 16%, 15%, 14%,12%,10%, 8%, 6%, 4%, 2% or 1%. Suitably the rate of decline in DLCO is about 5%, such as about 10%, such as about 15%, such as about 20%, such as about 25%.

In one embodiment, the IPF is slowly progressive IPF. In one embodiment, slowly progressive IPF is characterised by a rate of decline in FVC of greater than or equal to 1%, such as greater than or equal to 2%, 4%, 6% or 8%. In one embodiment, slowly progressive IPF is characterised by a rate of decline in FVC of about 5%, such as about 10%. In one embodiment, slowly progressive IPF is characterised by a rate of decline in DLCO of greater than or equal to 1%, such as greater than or equal to 2%, 4%, 6%, 8%, 10%, 12% or 14%. In one embodiment, rapidly progressive IPF is characterised by a rate of decline in DLCO of about 5%, such as about 10%.

In one embodiment, the IPF is rapidly progressive IPF. In one embodiment, rapidly progressive IPF is characterised by a rate of decline in FVC of greater than or equal to 10%, such as greater than or equal to 12%,14%, 16%, 18% or 20%. In one embodiment, rapidly progressive IPF is characterised by a rate of decline in FVC of about 15%, such as about 20%. In one embodiment, rapidly progressive IPF is characterised by a rate of decline in DLCO of greater than or equal to 15%, such as greater than or equal to 16%, 18%, 20%, 22% or 25%. In one embodiment, rapidly progressive IPF is characterised by a rate of decline in DLCO of about 15%, such as about 20% or 25%.

It is to be understood, that the IPF can be mild, moderate, mild-to-moderate, or severe, and rapidly or slowly progressive. Rapidly and slowly progressive relates to the rate of the progression of the IPF, while mild, moderate, mild-to-moderate and severe relate to what level the IPF has advanced. In one embodiment, the IPF is rapidly progressive IPF wherein the IPF is characterised as mild, moderate, mild-to-moderate or severe IPF. In one embodiment, the IPF is slowly progressive IPF wherein the IPF is characterised as mild, moderate, mild-to-moderate or severe IPF.

In another aspect, there is provided a saracatinib, or a pharmaceutically acceptable salt thereof, for use in treating rapidly and/or slowly progressive IPF wherein the IPF is characterised as mild, moderate, mild-to-moderate, or severe in a human patient.

### Safety Profile

In one embodiment, the saracatinib or a pharmaceutically acceptable salt thereof has a well-tolerated safety profile. That is, the saracatinib or a pharmaceutically acceptable salt thereof can be used in a dose to elicit efficacy while demonstrating safety and tolerability. In one embodiment, when used to treat IPF, the saracatinib or a pharmaceutically acceptable salt thereof, shows the same or improved efficacy with reduced adverse reactions or discontinuations as compared to pirfenidone or nintedanib.

Adverse reactions are classified by System Organ Class (SOC) into different frequency groupings. Very common refers to an incident rate of ≥1/10, common as ≥1/100 to <1/10, uncommon as ≥1/1000 to <1/100, and rare as ≥1/10000 to <1/1000. In one embodiment, the number of adverse reactions associated with the Src kinase inhibitor is classified as common, uncommon and/or rare.

In one embodiment, the adverse reactions are infections; infestations; blood disorders; lymphatic system disorders; immune system disorders; metabolism disorders; nutritional disorders; psychiatric disorders; nervous system disorders; vascular disorders; respiratory disorders; thoracic disorders; mediastinal disorders; gastrointestinal disorders; hepatobiliary disorders; skin disorders; subcutaneous tissue disorders; musculoskeletal disorders; connective tissue disorders; general disorders; administration site conditions; injury poisoning and/or procedural complications.

In one embodiment, the number of gastrointestinal adverse reactions associated with the saracatinib or a pharmaceutically acceptable salt thereof is classified as common, uncommon or rare. Suitably the gastrointestinal adverse reactions are diarrhoea, nausea, abdominal pain, dyspepsia, gastroesophageal reflux disease, vomiting, abdominal distension, abdominal discomfort, stomach discomfort, gastritis, constipation and/or flatulence.

In one embodiment, the number of hepatobiliary disorders associated with saracatinib or a pharmaceutically acceptable salt thereof is classified as common, uncommon or rare. Suitably the hepatobiliary disorder results in increased levels of hepatic enzyme in the human patient.

In one embodiment, the number of nutritional disorders associated with the saracatinib or a pharmaceutically acceptable salt thereof is classified as common, uncommon or rare. Suitably the nutritional disorder is anorexia.

In one embodiment, the number of skin disorders and/or subcutaneous tissue disorders associated with the saracatinib or a pharmaceutically acceptable salt thereof is classified as common, uncommon or rare. Suitably the skin disorders are a photosensitivity reaction and/or a rash.

In one embodiment, the number of general disorders associated with the saracatinib or a pharmaceutically acceptable salt thereof is classified as common, uncommon or rare. Suitably the general disorder is fatigue.

In one embodiment, the saracatinib or a pharmaceutically acceptable salt thereof has an improved safety profile to nintedanib and/or pirfenidone. In one embodiment, the saracatinib or a pharmaceutically acceptable salt thereof has a lower incidence rate and/or severity of adverse reactions relative to nintedanib and/or pirfenidone. In one embodiment, the saracatinib or a pharmaceutically acceptable salt thereof has a lower incidence rate and/or severity of gastrointestinal adverse reactions relative to nintedanib and/or pirfenidone. In one embodiment, the saracatinib or a pharmaceutically acceptable salt thereof has a lower incidence rate and/severity of diarrhoea, nausea, abdominal pain, dyspepsia, gastroesophageal reflux disease, vomiting, abdominal distension, abdominal discomfort, stomach discomfort, gastritis, constipation and/or flatulence relative to nintedanib and/or pirfenidone.

In one embodiment, administering to the human patient a therapeutically effective amount of saracatinib or a pharmaceutically acceptable salt thereof increases the overall and/or progression free survival rate of the human patient.

In one embodiment, administering to the human patient a therapeutically effective amount of saracatinib or a pharmaceutically acceptable salt thereof improves or prevent the symptoms of IPF, which include cough, lung function decline, fatigue and dyspnoea.

### Pharmaceutical compositions

According to a further aspect of the disclosure there is provided a pharmaceutical composition which comprises saracatinib or a pharmaceutically acceptable salt thereof as defined hereinbefore, in association with a pharmaceutically acceptable diluent, excipient or carrier for use in treating IPF as herein defined.

The compositions of the disclosure may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the disclosure may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more pharmaceutically acceptable diluents, excipients or carriers to produce a single dosage form will necessarily vary depending upon the host treated and the particular route of administration. For example, a formulation intended for oral administration to humans will generally contain, for example, from 0.5 mg to 0.5 g of active (more suitably from 0.5 to 200 mg, for example from 1 to 150 mg, 75 to 150 mg, 90 to 135 mg, 100 to 125 mg, such as 100 mg, such as about 125 mg) compounded with an appropriate and convenient amount of excipients. The size of the dose for therapeutic or prophylactic purposes of a Src kinase inhibitor will naturally vary according to the nature and severity of the conditions, the age sex of the animal or patient and the route of administration, according to well-known principles of medicine.

### Dosing regimen

In using saracatinib for therapeutic or prophylactic purposes it will generally be administered so that a daily dose in the range, for example, 0.1 mg/kg to 75 mg/kg body weight is received, given if required in divided doses. In general, lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. In one embodiment, saracatinib, is administered via oral administration, for example, in tablet or capsule form.

In one embodiment, the therapeutically effective amount of saracatinib is administered once daily, twice daily or three times daily. Suitably the therapeutically effective amount is administered once daily. Conveniently, the therapeutically effective amount is administered once daily for at least two consecutive days, suitably for at least or equal to 4 weeks, suitably for at least or equal to 14 weeks, suitably at least or equal to 26 weeks, suitably for at least or equal to 52 weeks, suitably for at least or equal to 2 years or longer.

A suitable dosage amount may be determined by reference to the severity of the disease and the size of the subject. Typical dosage amounts are in the range 0.01 mg to 500 mg, such as 0.1 to 175 mg, such as 1 to 125 mg per human dose to be delivered at least once daily, such as once or twice daily. For example, the dosage amounts for the saracatinib, can be 100 mg, 125 mg, or up to 250 mg per day.

In one embodiment, the therapeutically effective amount of the saracatinib is between 5 and 500 mg per day, suitably between 10 and 400 mg per day, suitably between 20 and 300 mg per day, suitably between 30 and 200 mg per day, suitably between 75 and 150 mg per day, suitably between 100 and 150 mg, suitably between 110 and 140 mg, suitably between 120 and 130 mg, or suitably about 125 mg.

### Combination therapy

In one embodiment, there is provided saracatinib or a pharmaceutically acceptable salt thereof for use in a method of treating IPF as described herein, comprising administering to the human patient a therapeutically effective amount of the saracatinib, or a pharmaceutically acceptable salt thereof, in combination with a therapeutically effective dose of at least one other therapeutic agent. The combined administrations can be separate, simultaneous, or sequential. In one embodiment, the IPF is characterised by increased expression of Collagen-I and/or MMP-9 and/or TIMP-1.

The therapeutic agent refers to a chemical or biological molecule, such a compound, peptide, nucleic acid, protein, and antibody, or fragments thereof, which can produce therapeutic effects in a human patient. In one embodiment, the therapeutic agent is a pharmaceutically active compound. In another embodiment, the therapeutic agent is an anti-fibrotic drug.

Such pharmacologically active compound may be compounds which are, for example, also pharmacologically active in the treatment of fibrosis, for example pirfenidone or nintedanib. Such pharmacologically active compounds may also be substances with a secretolytic, broncholytic and/or anti-inflammatory activity, such as anticholinergic agents, beta-2 mimetics, corticosteroids, PDE-IV inhibitors, p38 MAP kinase inhibitors, MK2 inhibitors, galectin inhibitors, NKi antagonists, LTD4 antagonists, EGFR inhibitors, VEGF inhibitors, PDGF inhibitors, FGF inhibitors, TGFbeta inhibitors, LPA1 antagonists, LOXL2 inhibitors, CTGF inhibitors, pentoxyfylline, N-acetylcysteine, anti-IL13 agents, anti IL4 agents, Alpha V integrin inhibitors (including inhibitors of αVβ1, αVβ2, αVβ3, αVβ4, αVβ5, αVβ6, αVβ7, αVβ8, and any combinations thereof), IGF inhibitors, PI3K inhibitors, mTOR inhibitors, JNK inhibitors, pentraxin2 and/or endothelin-antagonists.

Other pharmacologically active compounds to be used in combination with the saracatinib or a pharmaceutically acceptable salt thereof include compounds with an antifibrotic activity, such as PDE-III inhibitors, combined anti-IL4/13 agents, combined PI3k/mTOR inhibitors, autotaxin inhibitors, P2X2 antagonists, CTGF antagonists, 5-LO antagonists, leukotriene antagonists, ROCK inhibitors, PDGFR inhibitors (α and/or β), FGR inhibitors, and/or VEGFR inhibitors. In one embodiment, the anti-fibrotic drug is selected from pirfenidone, or a pharmaceutically acceptable salt thereof, or nintedanib, or a pharmaceutically acceptable salt thereof.

In one embodiment, there is provided saracatinib or a pharmaceutically acceptable salt thereof, for use in a method of treating fibrosis, wherein the method comprises administering to the human patient a therapeutically effective amount of the saracatinib, or a pharmaceutically acceptable salt thereof, and a therapeutically effective dose of at least one anti-fibrotic drug, either separately, simultaneously, sequentially, or in the form of a pharmaceutical composition comprising the saracatinib, or a pharmaceutically acceptable salt thereof, and the at least one anti-fibrotic. An anti-fibrotic drug is a drug, as herein defined, which when administered to a human patient, reduces and/or inhibits fibrosis.

In one embodiment, one anti-fibrotic drug is administered in combination with the saracatinib or a pharmaceutically acceptable salt thereof. In one embodiment, two anti-fibrotic drugs are administered in combination with the Src inhibitor or a pharmaceutically acceptable salt thereof.

It is to be understood that the anti-fibrotic drug may be in the free form (i.e., free acid or free base) or in the form of a pharmaceutically acceptable salt.

In one particular embodiment, the anti-fibrotic drug is selected from nintedanib or a pharmaceutically acceptable salt thereof; pirfenidone or a pharmaceutically acceptable salt thereof; and a combination thereof. In one embodiment, the anti-fibrotic drug is nintedanib or a pharmaceutically acceptable salt thereof. In one embodiment, the anti-fibrotic drug is pirfenidone or a pharmaceutically acceptable salt thereof. The present disclosure has surprisingly shown that saracatinib when combined with an anti-fibrotic drug such as nintedanib or pirfenidone, provide synergistic effect in the treatment of fibrosis.

In one embodiment, there is provided a pharmaceutical composition comprising the saracatinib, or pharmaceutically acceptable salt thereof, and nintedanib, or a pharmaceutically acceptable salt thereof, wherein, between 75 to 150 mg, 90 to 135 mg, or 100 to 125 mg of the saracatinib is present in the pharmaceutical composition.

### Kits

In a further aspect, there is provided a pharmaceutical combination comprising (a) saracatinib or a pharmaceutically acceptable salt thereof, and (b) a therapeutically effective amount of nintedanib or a pharmaceutically acceptable salt thereof; wherein saracatinib is present in an amount of 75 to 500 mg, such as 90 to 135 mg, such as 100 to 125 mg, such as about 100 mg, or such as about 125 mg. In one embodiment, saracatinib or a pharmaceutically acceptable salt thereof is in an oral dosage form for once per day administration.

In one embodiment, the pharmaceutical combination is a kit of parts. The kit comprises instructions for use and saracatinib, or a pharmaceutically acceptable salt thereof, as herein defined.

In a further aspect, there is provided a kit of parts, the kit comprising instructions for use and a pharmaceutical composition as herein defined. In one embodiment, the pharmaceutical composition comprises saracatinib, or a pharmaceutically acceptable salt thereof, and nintedanib, or pharmaceutically acceptable salt thereof.

### EXAMPLES

### Example 1 - Kinase selectivity

IC50 assay values for c-Src, c-Yes, Lck, Lyn, c-Fyn and EGFR kinases, and PGFRTKα and PGFRTKβ for Saracatinib, nintedanib and 4-amino-5-(4-chlorophenyl)-7-(dimethylethyl)pyrazolo[3,4-d]pyrimidine (PP2) are presented in Table 1 below.

The IC₅₀ assay values for saracatinib and PP2 are disclosed in Hennequin 2006 DOI:10.1021/jm060434q. The IC₅₀ assay values for nintedanib are disclosed in Hilberg et al, "Triple Angiokinase Inhibitor with Sustained Receptor Blockade and Good Antitumor Efficacy", Cancer Res 2008; 68: (12). June 15, 2008. DOI: 10.1158/0008-5472.CAN-07-6307 Published June 2008.

**Table 1: IC₅₀ assay results**

| **Kinase** | **Saracatinib (nM)** | **Nintedanib (nM)** | **PP2 (µM)** |
|---|---|---|---|
| **c-Src** | 2.7 | 156 | >10 |
| **c-Yes** | 4 | | |
| **Lck** | <4 | 16 | >100 |
| **Lyn** | 5 | 195 | .355 |
| **c-Fyn** | 10 | | .691 |
| **EGFR** | 66 | >50,000 | .403 |
| **PDGFRTKβ** | >5,000 | 59 | 1.53 |
| **PDGFRTKα** | 10,000 | 65 | >102 |

The IC₅₀ assay values presented in Table 1 show that saracatinib is potent and when compared to nintedanib or the compound PP2, is highly selective for the Src-family kinases (c-Src, c-Yes, Lck, Lyn and c-Fyn).

### Example 2 - Bleomycin Model

Bleomycin Sulfate was obtained from Sigma-Aldrich. The IU conversion to enzyme units (U) is approximately 1.5-2 U/mg. For delivery of 4 U/kg in 50 µL, a solution of 2 mg/mL was prepared in 0.9% NaCl. Bleomycin or vehicle was dosed on Day 0 intranasally. Compounds were dosed twice daily by oral gavage starting at 7 days post bleomycin induction timed to coincide with the historical start of fibrosis (pirfenidone at 200 mg/kg BID, nintedanib at 30 mg/kg BID, saracatinib at 1, 3 or 10 mg/kg BID). Body weights and clinical observations were recorded on days 7, 14, and 21. On day 21, mice were euthanized by cervical dislocation. One side of lung tissue was dissected and analyzed for hydroxyproline levels as an indicator of local collagen deposition, and the other side lung was fixed in Formalin for histopathology (Quantitative staining with sirius red). Half of the animals from each group were dosed 1 hour prior to terminal blood draw (see below) on Day 21 (for Cₘₐₓ); the other half of the animals were not dosed (Cₘᵢₙ) (Table 2).

**Table 2. Pharmacokinetics (PK) of Saracatinib**

| **Dose (mg/kg BID)** | **Cmax (nM) +/- SD** | **Cmin (nM) +/- SD** |
|---|---|---|
| 10 | 390 +/- 224 | 31.8 +/- 15.9 |
| 3 | 139 +/- 75 | 8.41 +/- 2.9 |
| 1 | 71.5 +/- 24 | 2.52 +/- 0.8 |

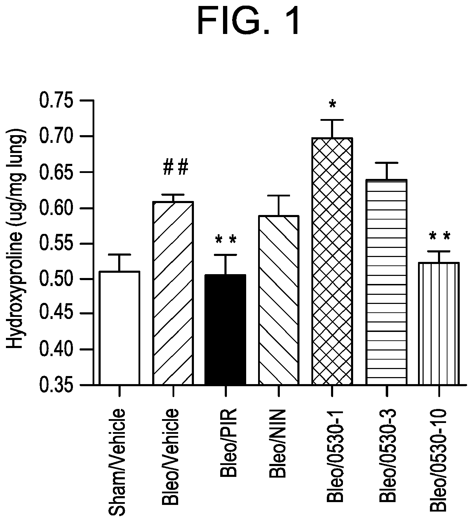

Results are presented in FIG. 1. The observed effects indicate that saracatinib compares favourably to pirfenidone (at much lower doses) and is potentially superior to nintedanib at clinically relevant doses.

### Example 3 - Effects on TGF-beta-induced changes in primary human lung fibroblasts from a healthy donor

Normal human lung fibroblasts from a healthy donor (passage 5) were seeded in 96-well culture plates with 50,000 cells per cm² in DMEM culture medium with 10% FBS and 1% penicillin-streptomycin. They were cultured at 37°C for 24 hours and then washed twice with DPBS and serum-starved for 24 hours before treatment was added in the form of saracatinib, nintedanib, pirfenidone or the positive control SB-525334, an Alk-5 inhibitor, or 0.1% DMSO as a vehicle control. The cells were incubated for one hour before 0.123ng/ml TGF-beta1 was added on top of the compound/vehicle treatment and the cells were incubated for 24 hours.

After 24 hours the medium was collected and analysed for IL-6 and the cells were either lysed for RNA isolation, DNA-synthesis and TaqMan quantitative PCR or fixated and stained for alpha-smooth muscle actin (αSMA) and with Hoechst nuclear staining. The percentage of cells staining positive for αSMA and thereby displaying the myofibroblast phenotype was determined using high content image analysis. The Ct-values from the quantitative PCR were normalised against the geomean Ct values of two reference-genes and presented as fold-expression over those reference genes (2^-delta Ct).

The doses needed to inhibit 50% of the TGF-beta1-induced response (IC50) were determined for the different compounds by fitting dose-response-curves to the data using a four-parameter non-linear regression model.

**Table 3: Mean IC₅₀ values generated from TGFβ-stimulated NHLF experiments performed in using healthy donor 197 (n=3)**

| **Treatment** | **αSMA** | **IL-6 protein** | **ACTA2 mRNA** | **COL1A1 mRNA** | **COL3A1 mRNA** | **FN1 mRNA** |
|---|---|---|---|---|---|---|
| Saracatinib | 185 nM | 128 nM | 186 nM | 1010 nM | 7990 nM | 526 nM |
| Pirfenidone | >10uM | >10uM | >10uM | >10uM | >10uM | >10uM |
| Nintedanib | 601 nM | 311 nM | 1750 nM | 536 nM | 725 nM | 624 nM |

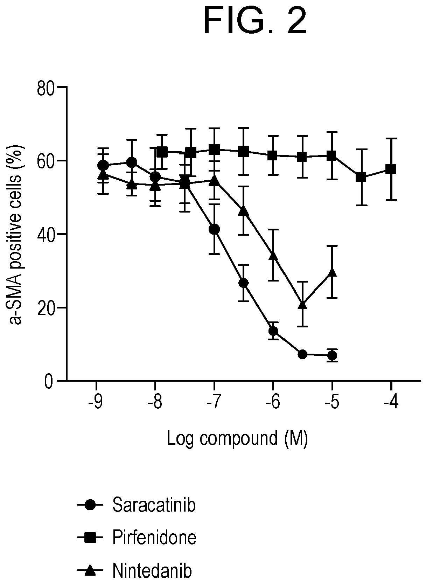

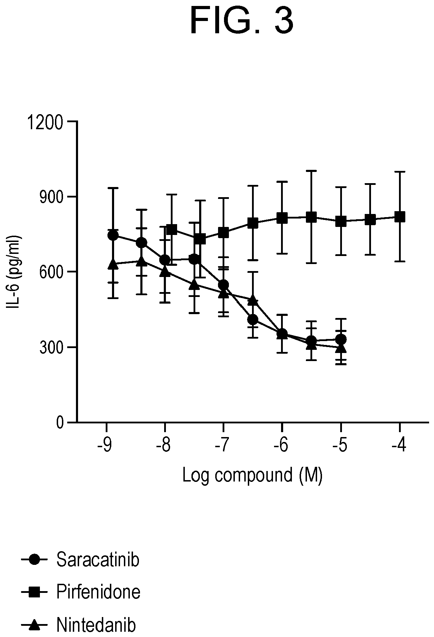

Saracatinib showed superior potency at therapeutic concentrations (Table 3, FIG. 2 and FIG. 3). Indeed, saracatinib caused dose-dependent inhibition of αSMA without impacting cell viability. Nintedanib and pirfenidone inhibited αSMA, but at supra-therapeutic concentrations and with some evidence of cell death.

### Example 4 - Organoid formation assay

Airway basal cells (ABC) from IPF patients have been described as "cancer like" in their proliferation, migration, and resistance to apoptosis. They are enriched in areas of bronchiolization, where they extend into fibroblastic foci; structures directly linked to lung function. Healthy control (HC)-ABC can be stimulated to produce 3D organoids in culture with growth factors and cytokines. However, IPF-ABC spontaneously form 3D organoids in culture. Following on to the success observed in oncology, IPF-ABC organoid formation is being explored as a tool to screen for therapeutic activity (A Prasse et al., European Respiratory Journal 2017; 50; and A Prasse et al., Am J Respir Crit Care Med. 2018 Aug 24. doi: 10.1164/rccm.201712-2551OC).

ABC is understood to be relevant to IPF pathology and the signal observed correlates to disease severity (FVC decline, need for lung transplant, and risk of mortality). Indeed, ABC models reproduce the aberrant wound healing in IPF (*e.g.* formation of fibroblast tubules, 3D organoid structures, production of ECM components). Mice transplanted with human IPF ABC recapitulate broad spectrum of IPF pathology, including robust fibrotic response, migration, invasion of alveolar compartment, cystic structures, etc.

Airway Basal Cells (ABCs) either derived from IPF patients or Healthy Volunteers (HV) were cultured in Matrigel^{®} (Corning in a transwell system with or without lung fibroblasts either derived from patients with IPF or normal lung for up-to 35 days in the incubator (5% CO₂, 37°C)). Medium exchange (BEGM (Lonza, Basel, Switzerland, #CC-3170)/ DMEM (Dulbecco's Modified Eagle Medium (Gibco, Swit Fisher Scientific/Germany); ratio 1:1) was done every 7 days. The conditioned medium was used for Sircol assay (Scientific-Biocolor/UK, # S1000) which was performed as recommended by the manufacturer (https://www.biocolor.co.uk/product/sircol-soluble-collagen-assay/). The organoids were treated with 8, 25, 75, 210 or 600 nM saracatinib, or pirfenidone at 1 mM or nintedanib at 1 µM (pirfenidone and nintedanib acting as positive controls). Spheroid formation was documented by Axio Vert.A1/Zeiss/Germany and Axio Observer Z1/Zeiss/Germany.

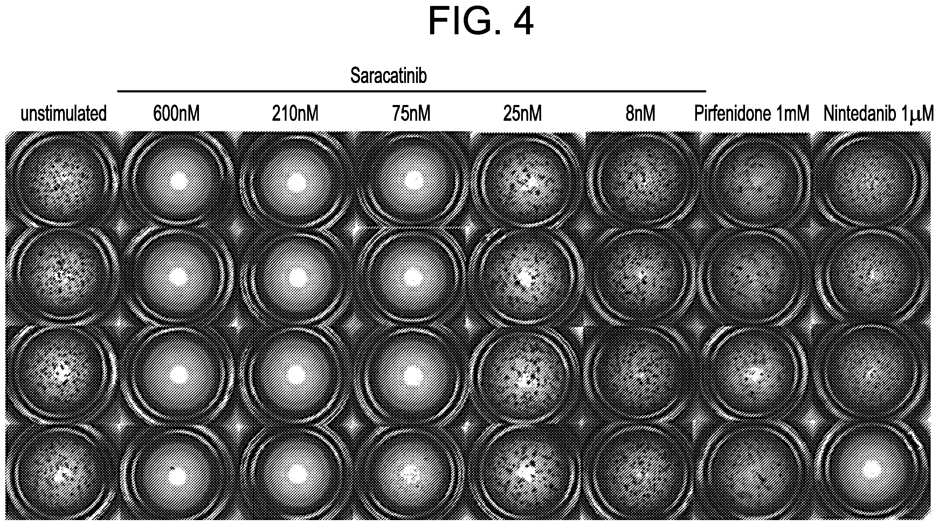

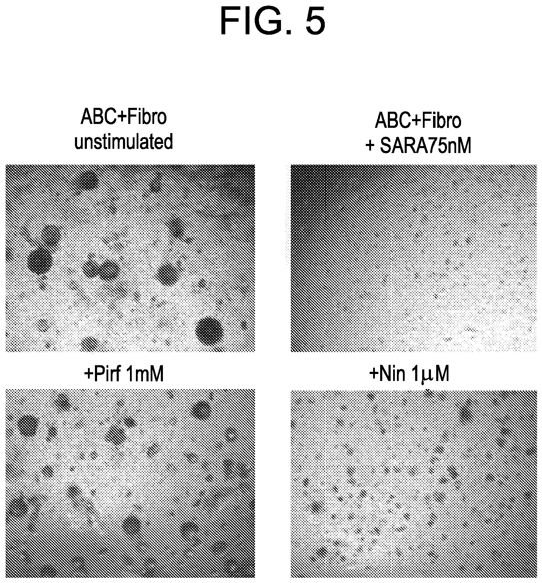

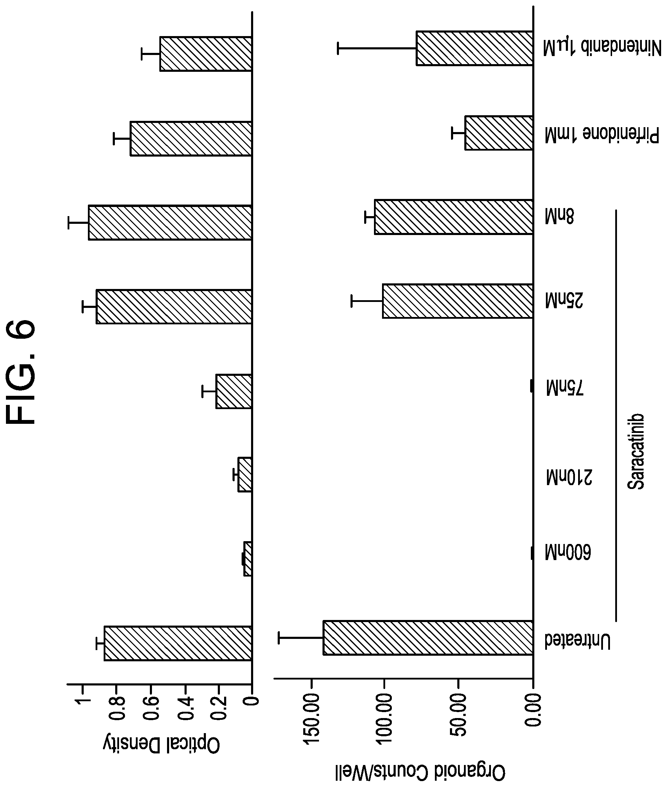

The results are presented in Figures 4, 5 and 6. Figures 4 and 5 show spheroid formation of ABCs treated with saracatinib, pirfenidone or nintedanib at different magnification levels. Saracatinib is non-cytotoxic at the concentrations tested. Nintedanib and pirfenidone at artificially high, supratherapeutic doses have little to no effect on organoid formation. These data in the human IPF ABC mouse model suggest saracatinib is differentiated and uniquely efficacious relative to nintedanib or pirfenidone.

### Example 5 - DiscoverX

BioMAP panels consist of human primary cell-based systems designed to model different aspects of the human body in an in vitro format. The 12 systems available in the Diversity PLUS panel allow test agent characterization in an unbiased way across a broad set of systems modelling various human disease states. BioMAP systems are constructed with one or more primary cell types from healthy human donors, with stimuli (such as cytokines or growth factors) added to capture relevant signalling networks that naturally occur in human tissue or pathological conditions. Systems recapitulate aspects of the systemic immune response including monocyte-driven Th1 inflammation (LPS system) or T cell stimulation (SAg system), chronic Th1 inflammation driven by macrophage activation (IMphg system) and the T cell-dependent activation of B cells that occurs in germinal centers (BT system). The BE3C system (Th1) and the BF4T system (Th2) represent airway inflammation of the lung, while the MyoF system models myofibroblast-lung tissue remodelling.

Each test agent generates a signature BioMAP profile that is created from the changes in protein biomarker readouts within individual system environments. Biomarker readouts (7 - 17 per system) are selected for therapeutic and biological relevance, are predictive for disease outcomes or specific drug effects and are validated using agents with known mechanism of action (MoA). Each readout is measured quantitatively by immune-based methods that detect protein (e.g., ELISA) or functional assays that measure proliferation and viability. BioMAP readouts are diverse and include cell surface receptors, cytokines, chemokines, matrix molecules and enzymes.

Using custom-designed software containing data mining tools, a BioMAP profile can be compared against a proprietary reference database of > 4,000 BioMAP profiles of bioactive agents (biologics, approved drugs, chemicals and experimental agents) to classify and identify the most similar profiles. This robust data platform allows rapid evaluation and interpretation of BioMAP profiles by performing the unbiased mathematical identification of similar activities. Specific BioMAP activities have been correlated to in vivo biology, and multiparameter BioMAP profiles have been used to distinguish compounds based on MoA and target selectivity and can provide a predictive signature for in vivo toxicological outcomes (e.g., vascular toxicity, developmental toxicity, etc.) across diverse physiological systems.

Saracatinib and nintedanib were prepared as stock solutions at a 10 mM concentration in 100% DMSO. In the Combo ELECT panel, a 4x4 combination array is created to test all mixtures of two serially diluted single test agents/drugs. Test agents/drugs are screened at 4 concentrations each (3-fold dilutions: saracatinib at 3.3, 1.1, 0.367, and 0.123 µM; nintedanib at 1, 330, 110, and 37 µM), in triplicate, in selected BioMAP Systems (50+ human biology and disease model systems available through BioMAP Combo ELECT).

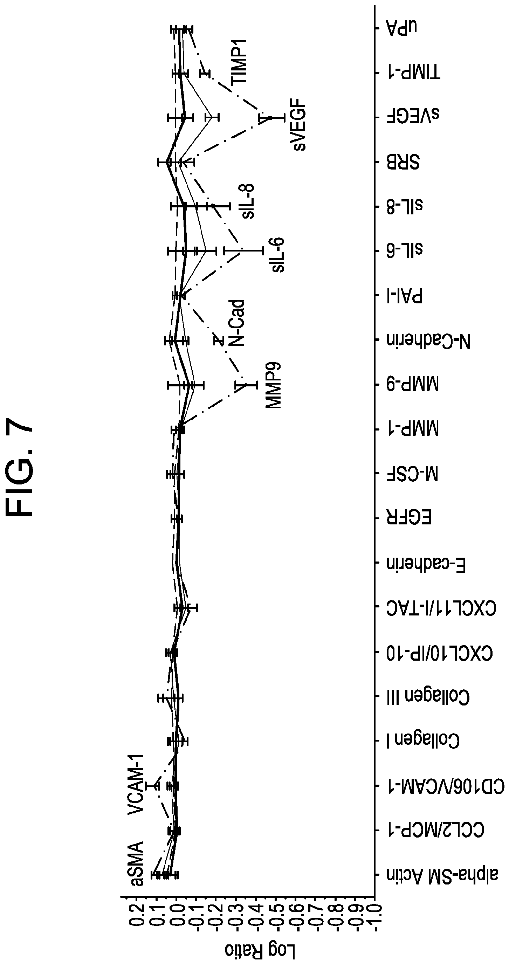

The results are shown in Figures 7, 8 and 9.

FIG. 7 shows that saracatinib causes decreased expression of N-cadherin and increased expression of α-SMA; both of which are associated with myofibroblast activation. Saracatinib also causes decreased expression of TIMP-1 and MMP-9; both of which are associated with fibrosis-related matrices. Saracatinib causes decreased expression of sVEGF. sVEGF expression is associated with tissue remodelling and/or wound healing. Additionally, saracatinib causes decreased expression of sIL-8 and sIL-6 and increased expression of VCAM-1 proteins. These are associated with inflammation.

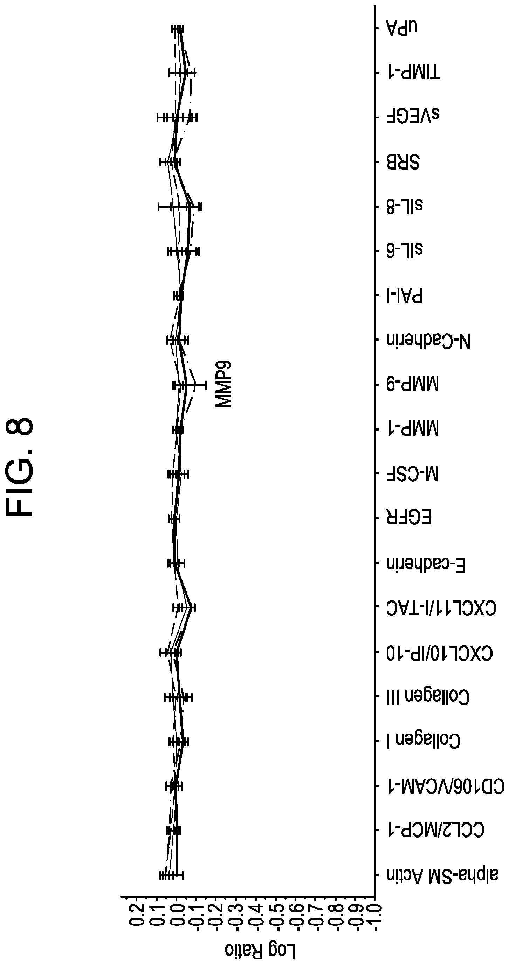

FIG. 8 shows that nintedanib causes decreased expression if TIMP-1 and MMP-9; both of these are associated with fibrosis-related matrices.

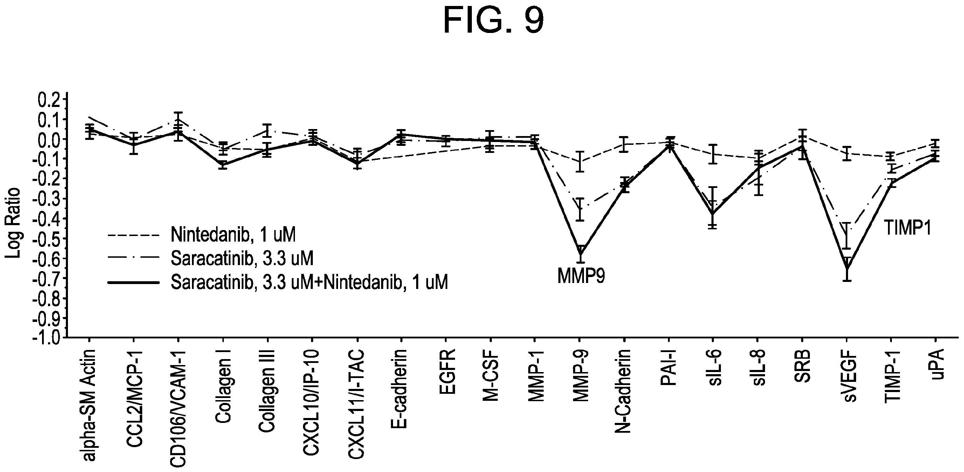

FIG. 9 shows that the combination of saracatinib and nintedanib causes novel inhibition of Collagen-I, and greater inhibition of MMP-9 and TIMP-1 relative to saracatinib or nintedanib alone.

### Example 6 - Safety and Tolerability

Saracatinib (100 mg or 125 mg per day) had been tested in double-blind placebo-controlled clinical trials in human patients suffering Alzheimer's Disease (AD). The safety and tolerability results are summarized in Tables 4, 5, and 6 below in comparison with the safety and tolerability profiles of pirfenidone (Noble et al 2016 doi: 10.1183/13993003.00026-2015) and nintedanib (Richeldi et al 2016. doi: 10.1016/j.rmed.2016.02.001) for treating idiopathic pulmonary fibrosis (IPF).

**Table 4. Gastrointestinal Disorders**

| | Pirfenidone IPF | | Nintedanib IPF | | Saracatinib AD | |
|---|---|---|---|---|---|---|
| | Pivotal Studies (52 weeks) | | Pivotal Studies (52 weeks) | | Phase Ila (52 weeks) | |
| Gastrointestinal Events | Pirfenidone | Placebo | Nintedanib | Placeb o | Saracatini b | Placebo |
| | n=623 | n= 624 | n= 723 | n=508 | n=79 | n=80 |
| Nausea | 35.5% | 15.1% | 24.3% | 7.1% | 12.7% | 8.8% |
| Vomiting | 12.7% | 6.1% | 11.8% | 3.0% | 5.1% | 6.3% |
| Diarrhea | 24.6% | 18.8% | 61.5% | 17.9% | 27.8% | 11.3% |

As shown by Table 4, saracatinib has a better gastrointestinal tolerability profile than nintedanib.

**Table 5. General Disorders**

| | Pirfenidone IPF Pivotal Studies (52 weeks)¹ | | Nintedanib IPF Pivotal Studies (52 weeks)² | | Saracatinib AD Phase Ila (52 weeks) | |
|---|---|---|---|---|---|---|
| General Disorders | Pirfenidone | Placebo | Nintedanib | Placebo | Saracatinib | Placebo |
| | n=623 | n= 624 | n= 723 | n=508 | n=79 | n=80 |
| Anorexia | 13% | 5% | NA | NA | 3% | 0% |
| Decreased Appetite | 9%³ | 3%³ | 11% | 5% | 9% | 1% |
| Weight Decrease | 10% | 5% | 10% | 3% | 10% | 5% |

| | | | | | | |
|---|---|---|---|---|---|---|
| ¹Nobel et al 2016 ²Richeldi et al 2016 ³Pirfenidone Medical Review (pirfenidone n=345; placebo n=347) | | | | | | |

As shown by Table 5, saracatinib has a similar or better general tolerability profile than nintedanib and pirfenidone.

**Table 6. Treatment Discontinuations**

| Nintedanib | | Pirfenidone | | Saracatinib | |
|---|---|---|---|---|---|
| Clinical studies IPF (Placebo) | Real-World data | Clinical studies IPF (Placebo) | Real-World data | Clinical studies AD (Placebo) | Real-World data |
| 21% (15%) | 26.3% | 14.6% (9.6%) | 20.9% | 8.2% (1.2%) | NA |

As shown by Table 6, saracatinib treatment may result in less discontinuations.

This written description uses examples to disclose the invention and to enable any person skilled in the art to practice the invention, including making and using any of the disclosed salts, substances, or compositions, and performing any of the disclosed methods or processes.

## Claims

1. Saracatinib or a pharmaceutically acceptable salt thereof for use in the treatment of Idiopathic Pulmonary Fibrosis (IPF) in a human patient, wherein the IPF is **characterized by** abnormal collagen deposition, or by persistent, debilitating abnormal formation of lesions or scars determined by high-resolution computed tomography (HRCT) or biopsy.

2. Saracatinib or a pharmaceutically acceptable salt thereof for use according to claim 1, wherein the IPF is further **characterized by** epithelial to mesenchymal transition (EMT).

3. Saracatinib or a pharmaceutically acceptable salt thereof for use according to claim 1 or 2, wherein the IPF is further **characterized by** increased expression of Collagen-I and/or MMP-9 and/or TIMP-1.

4. Saracatinib or a pharmaceutically acceptable salt thereof for use according to any one of claims 1 to 3, wherein the IPF is further **characterized by** increased expression of sVEGF and/or sIL-8 and/or sIL-6.

5. Saracatinib or a pharmaceutically acceptable salt thereof for use according to any one of claims 1 to 3, wherein the IPF is further **characterized by** decreased expression of VCAM-1.

6. Saracatinib or a pharmaceutically acceptable salt thereof for use according to any one of claims 1 to 3, wherein the IPF is further **characterized by** extra cellular matrix (ECM) formation.

7. Saracatinib or a pharmaceutically acceptable salt thereof for use in treatment of Idiopathic pulmonary fibrosis in a human patient, wherein the Saracatinib or a pharmaceutically acceptable salt thereof is administered in combination with a therapeutically effective amount of nintedanib or a pharmaceutically acceptable salt thereof.

8. Saracatinib or a pharmaceutically acceptable salt thereof for use according to claim 7, wherein the IPF is **characterized by** increased expression of Collagen-I and/or MMP-9 and/or TIMP-1.

9. A pharmaceutical combination comprising:
a. saracatinib or a pharmaceutically acceptable salt thereof; and
b. a therapeutically effective amount of nintedanib or a pharmaceutically acceptable salt thereof;
wherein saracatinib is present in an amount of 75 to 500 mg, such as 90 to 135 mg, such as 100 to 125 mg, such as about 100 mg, or such as about 125 mg.

10. The pharmaceutical combination according to claim 9, wherein saracatinib or a pharmaceutically acceptable salt thereof is in an oral dosage form for once per day administration.

## Patentansprüche

1. Saracatinib oder pharmazeutisch verträgliches Salz davon zur Verwendung bei der Behandlung von idiopathischer Lungenfibrose (IPF) bei einem menschlichen Patienten, wobei die IPF **gekennzeichnet ist durch** anormale Kollagenablagerung oder durch persistierende, belastende anormale Bildung von Läsionen oder Narben, festgestellt durch hochauflösende Computertomographie (HRCT) oder Biopsie.

2. Saracatinib oder pharmazeutisch verträgliches Salz davon zur Verwendung nach Anspruch 1, wobei die IPF ferner **gekennzeichnet ist durch** epithelial-mesenchymale Transition (EMT).

3. Saracatinib oder pharmazeutisch verträgliches Salz davon zur Verwendung nach Anspruch 1 oder 2, wobei die IPF ferner **gekennzeichnet ist durch** erhöhte Expression von Kollagen-I und/oder MMP-9 und/oder TIMP-1.

4. Saracatinib oder pharmazeutisch verträgliches Salz davon zur Verwendung nach einem der Ansprüche 1 bis 3, wobei die IPF ferner **gekennzeichnet ist durch** erhöhte Expression von sVEGF und/oder sIL-8 und/oder sIL-6.

5. Saracatinib oder pharmazeutisch verträgliches Salz davon zur Verwendung nach Anspruch 1 oder 3, wobei die IPF ferner **gekennzeichnet ist durch** verminderte Expression von VCAM-1.

6. Saracatinib oder pharmazeutisch verträgliches Salz davon zur Verwendung nach einem der Ansprüche 1 bis 3, wobei die IPF ferner **gekennzeichnet ist durch** Bildung von extrazellulärer Matrix (ECM).

7. Saracatinib oder pharmazeutisch verträgliches Salz davon zur Verwendung bei der Behandlung von idiopathischer Lungenfibrose bei einem menschlichen Patienten, wobei das Saracatinib oder ein pharmazeutisch verträgliches Salz davon in Kombination mit einer therapeutisch wirksamen Menge von Nintedanib oder einem pharmazeutisch verträglichen Salz davon verabreicht wird.

8. Saracatinib oder pharmazeutisch verträgliches Salz davon zur Verwendung nach Anspruch 7, wobei die IPF **gekennzeichnet ist durch** erhöhte Expression von Kollagen-I und/oder MMP-9 und/oder TIMP-1.

9. Pharmazeutische Kombination, umfassend:
a. Saracatinib oder ein pharmazeutisch verträgliches Salz davon, und
b. eine therapeutisch wirksame Menge Nintedanib oder ein pharmazeutisch verträgliches Salz davon,
wobei Saracatinib in einer Menge von 75 bis 500 mg, wie 90 bis 135 mg, wie 100 bis 125 mg, wie etwa 100 mg oder wie etwa 125 mg vorhanden ist.

10. Pharmazeutische Kombination nach Anspruch 9, wobei Saracatinib oder ein pharmazeutisch verträgliches Salz davon in einer oralen Darreichungsform zur einmal täglichen Verabreichung vorliegt.

## Revendications

1. Saracatinib ou sel pharmaceutiquement acceptable correspondant pour une utilisation dans le traitement d'une fibrose pulmonaire idiopathique (IPF) chez un patient humain, l'IPF étant **caractérisée par** un dépôt anormal de collagène, ou par la formation anormale débilitante persistante de lésions ou de cicatrices déterminées par tomodensitométrie à haute résolution (HRCT) ou une biopsie.

2. Saracatinib ou sel pharmaceutiquement acceptable correspondant pour une utilisation selon la revendication 1, l'IPF étant en outre **caractérisée par** une transition d'épithéliale à mésenchymateuse (EMT) .

3. Saracatinib ou sel pharmaceutiquement acceptable correspondant pour une utilisation selon la revendication 1 ou 2, l'IPF étant en outre **caractérisée par** une expression augmentée de Collagène-I et/ou MMP-9 et/ou TIMP-1.

4. Saracatinib ou sel pharmaceutiquement acceptable correspondant pour une utilisation selon l'une quelconque des revendications 1 à 3, l'IPF étant en outre **caractérisée par** une expression augmentée de sVEGF et/ou sIL-8 et/ou sIL-6.

5. Saracatinib ou sel pharmaceutiquement acceptable correspondant pour une utilisation selon l'une quelconque des revendications 1 à 3, l'IPF étant en outre **caractérisée par** une expression diminuée de VCAM-1.

6. Saracatinib ou sel pharmaceutiquement acceptable correspondant pour une utilisation selon l'une quelconque des revendications 1 à 3, l'IPF étant en outre **caractérisée par** la formation d'une matrice extracellulaire (ECM).

7. Saracatinib ou sel pharmaceutiquement acceptable correspondant pour une utilisation dans le traitement d'une fibrose pulmonaire idiopathique chez un patient humain, le saracatinib ou un sel pharmaceutiquement acceptable correspondant étant administré en combinaison avec une quantité thérapeutiquement efficace de nintédanib ou d'un sel pharmaceutiquement acceptable correspondant.

8. Saracatinib ou sel pharmaceutiquement acceptable correspondant pour une utilisation selon la revendication 7, l'IPF étant **caractérisée par** une expression augmentée de Collagène-I et/ou MMP-9 et/ou TIMP-1.

9. Combinaison pharmaceutique comprenant :
a. du saracatinib ou un sel pharmaceutiquement acceptable correspondant ; et
b. une quantité thérapeutiquement efficace de nintédanib ou d'un sel pharmaceutiquement acceptable correspondant ;
le saracatinib étant présent en une quantité de 75 à 500 mg, telle que de 90 à 135 mg, telle que de 100 à 125 mg, telle que d'environ 100 mg, ou telle que d'environ 125 mg.

10. Combinaison pharmaceutique selon la revendication 9, le saracatinib ou un sel pharmaceutiquement acceptable correspondant étant dans une forme posologique orale pour une administration une fois par jour.
